# EUROPEAN PATENT SPECIFICATION

(11) **EP 1 564 306 B1**
(45) Date of publication and mention of the grant of the patent: **07.08.2013**
(21) Application number: 05250915.5
(22) Date of filing: 17.02.2005
(51) Int. Cl.: C12Q 1/68

(54) **Methods for fragmenting and labeling DNA**
Verfahren zur Fragmentierung und Markierung von DNS
Procédé de fragmentation et de marquage d'ADN

(30) Priority: 17.02.2004 US 545417 P; 20.07.2004 US 589648 P; 06.10.2004 US 616652 P; 22.12.2004 US 639193 P
(43) Date of publication of application: 17.08.2005
(73) Proprietor: Affymetrix, Inc., Santa Clara, CA 95051 (US)
(72) Inventor: McGall, Glenn H., Palo Alto, CA 94303 (US); Barone, Anthony D., San Jose, CA 95125 (US); Chen, Chuan, Santa Clara, CA 95051 (US)
(74) Representative: Bizley, Richard Edward

(56) References cited:
- WO-A-00/24939
- WO-A-02/090584
- WO-A-2004/007751
- WO-A-2004/011665
- WO-A-2005/045060
- PROUDNIKOV D ET AL: "Chemical methods of DNA and RNA fluorescent labeling" NUCLEIC ACIDS RESEARCH, OXFORD UNIVERSITY PRESS, SURREY, GB, vol. 24, no. 22, 1996, pages 4535-4542, XP002255070 ISSN: 0305-1048
- LHOMME JEAN ET AL: "Abasic DNA structure, reactivity, and recognition" BIOPOLYMERS, vol. 52, no. 2, 1999, pages 65-83, XP002331444 ISSN: 0006-3525
- WODICKA ET AL: "GENOME-WIDE EXPRESSION MONITORING IN SACCHAROMYCES CEREVISIAE" NATURE BIOTECHNOLOGY, NATURE PUBLISHING, US, vol. 15, December 1997 (1997-12), pages 1359-1367, XP002100297 ISSN: 1087-0156

## Description

### RELATED APPLICATIONS

This application claims priority to U.S. Provisional Patent Application Nos. 60/545,417 filed February 17, 2004, 60/639,193 filed December 22, 2004, 60/616,652 filed October 6, 2004 and 60/589,648 filed July 20, 2004.

### FIELD OF THE INVENTION

Methods for fragmenting DNA using a chemical nuclease are disclosed. Methods for labelling the fragmented samples are also disclosed. Methods for detection of nucleic acids on a nucleic acid array are also disclosed.

### BACKGROUND OF THE INVENTION

Nucleic acid sample preparation methods have greatly transformed laboratory research that utilize molecular biology and recombinant DNA techniques and have also impacted the fields of diagnostics, forensics, nucleic acid analysis and gene expression monitoring, to name a few. There remains a need in the art for methods for reproducibly and efficiently fragmenting nucleic acids used for hybridization to oligonucleotide arrays.

### SUMMARY OF THE INVENTION

Methods and compositions are provided for fragmenting nucleic acid samples. In one aspect the invention provides a method for fragmenting and labeling DNA comprising: mixing the DNA in a reaction comprising a buffer that has a pH between 6 and 9 at a first temperature, wherein said first temperature is between 16 and 37°C and less than 6 at a second temperature, wherein said second temperature is between 65 and 105°C, wherein the reaction is mixed at said first temperature; incubating the reaction at the second temperature to generate a plurality of abasic sites in the DNA; incubating the reaction under conditions that promote cleavage of abasic sites and optionally with a nuclease that has 3' phosphatase activity, wherein the condition that promotes cleavage of abasic sites comprises incubation with an apurinic/apyrimidinic (AP) endonuclease; and, labeling the fragments in a reaction comprising TdT; wherein the buffer comprises a buffer selected from the group consisting of Tris, imidazole and colamine. In another aspect the invention provides a method for fragmenting and labeling a nucleic acid sample comprising DNA comprising: generating a plurality of abasic sites in the DNA by a chemical method; cleaving the phosphate backbone at a plurality of the abasic sites; optionally removing modifications at the 3' ends of the fragments, wherein said modifications are moieties other than a 3' hydroxyl group; and labeling the fragments with a detectable label; wherein the nucleic acid sample is in a buffer solution comprising a buffer selected from the group consisting of Tris, imidazole and colamine and wherein said buffer solution has a pH between 6 and 9 at a temperature between 20 and 30°C and a pH less than 6 at a temperature greater than 85°C and wherein said chemical method comprises incubating the sample at a temperature greater than 85°C for at least 15 minutes. In preferred embodiments, the methods and compositions are used to fragment DNA samples for labeling and hybridization to oligonucleotide arrays. The methods may be used, for example, for gene expression monitoring and for genotyping.

In some aspects the DNA that is to be fragmented is an amplification product. In a preferred embodiment the DNA is cDNA that is an amplification product of a sample containing RNA transcripts. RNA transcript samples may be used as templates for reverse transcription to synthesize single stranded cDNA or double stranded cDNA. Methods for cDNA synthesis are well known in the art. The resulting cDNA may be used as template for in vitro transcription to synthesize cRNA and the cRNA may then be used as template for additional cDNA synthesis as described in U.S. Patent Application No. 10/917,643. The resulting cDNA may be single or double stranded.

In one aspect the DNA sample to be fragmented is in an aqueous solution containing a buffer that is neutral (pH greater than or equal to 6.0) at a temperature between 20 and 37°C but becomes acidic (pH less than 6.0) at a temperature between 80 and 105°C. The buffer is a Tris (Tris(hydroyxmethyl)aminomethane) buffer solution, an imidazole buffer solution or a colamine buffer solution. The heating results in acidic conditions that generate abasic sites in the DNA by acid catalyzed depurination. The abasic sites can subsequently be cleaved thermally, by base treatment or by the use of an endonuclease that recognizes and cleaves abasic sites, for example Endo IV or Ape 1. Following cleavage at the abasic sites the fragments may be end labeled by terminal transferase to incorporate a detectable label into the 3' end of the fragments. In some aspects the abasic fragments are cleaved thermally or chemically and the 3' ends may be blocked from enzymatic labeling and the fragments may be treated with an AP endonuclease to remove blocking modifications prior to TdT labeling. The detectable label may include, for example, one or more biotins.

In another aspect the depurinated DNA is fragmented by chemical or thermal treatment and the fragments are chemically labeled. Chemical labeling may be by reaction with RNH₂ where R is the detectable label. In a preferred aspect R is biotin.

### BRIEF DESCRIPTION OF THE FIGURES

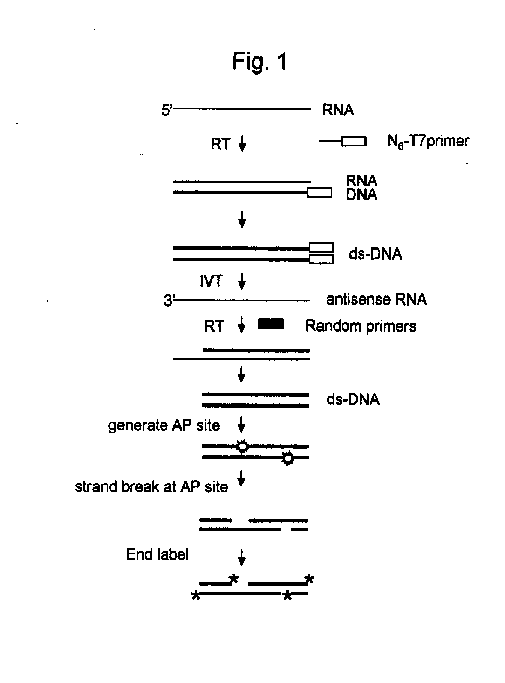

Figure 1 shows a schematic of a whole transcript amplification, fragmentation and labeling method.

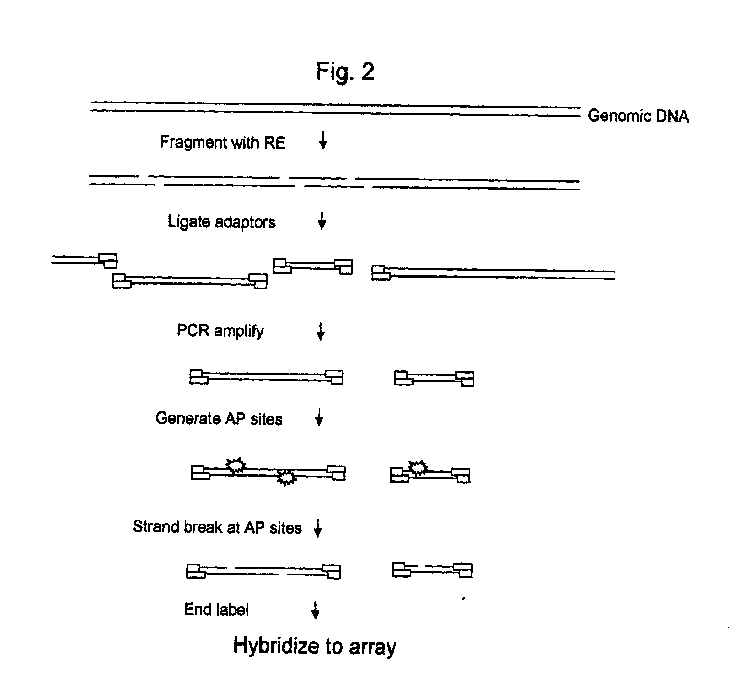

Figure 2 shows a schematic of a method of amplifying and reducing the complexity of a genomic DNA sample followed by fragmentation and labeling of the amplification products.
Figure 3 shows fragmentation by acid-catalzyed depurination. Abasic sites and 3' modified fragments are generated.
Figure 5 shows propose mechanisms and distribution of products for oxidative scission. Oxidation at different sites of the deoxyribose leads to different 3' modified ends that may require further treatment to generate ends suitable for TdT end labeling.

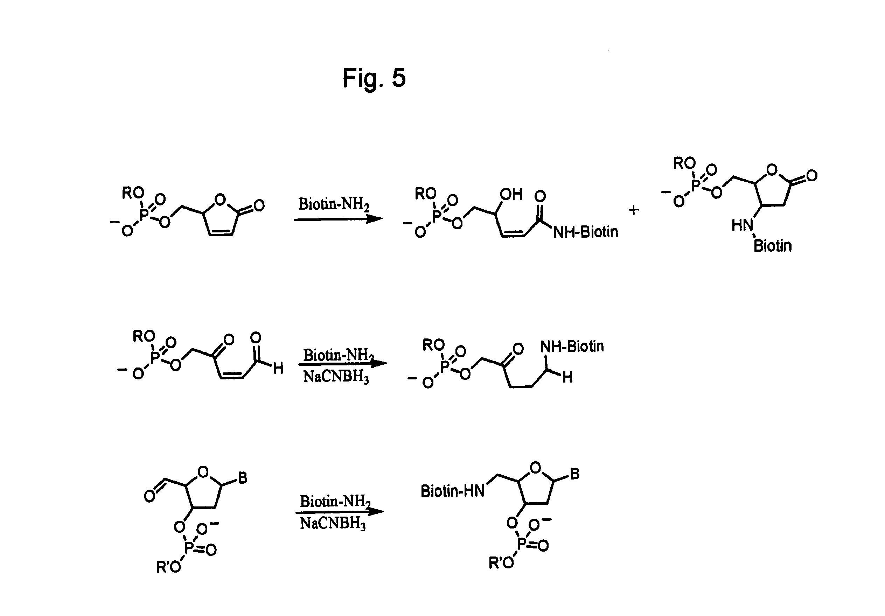

Figure 5 shows chemical labeling of oxidative scission products by reductive amination with RNH2.

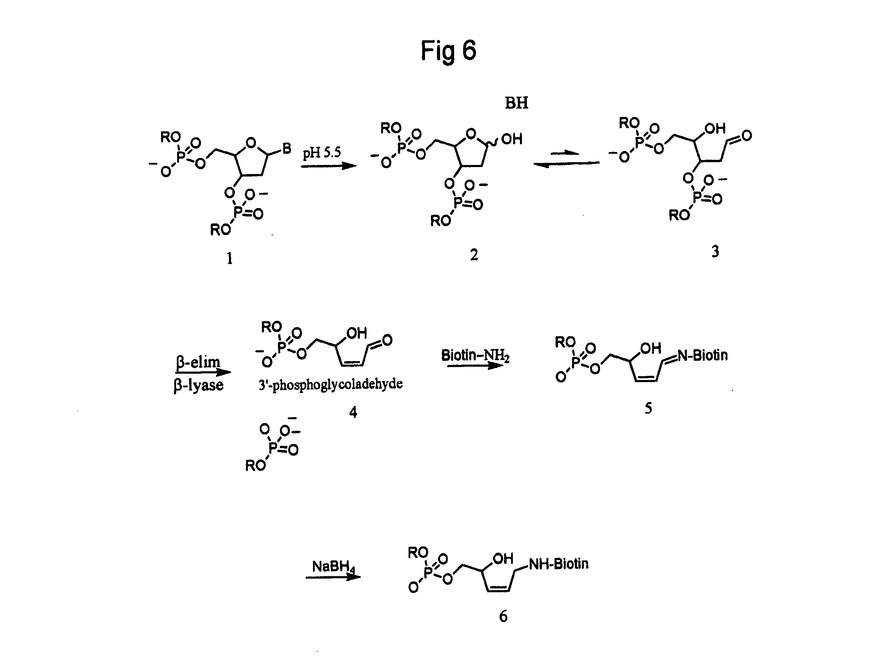

Figure 6 shows a method of cleaving depurinated DNA using a β-lyase followed by labeling with a biotin-amine.

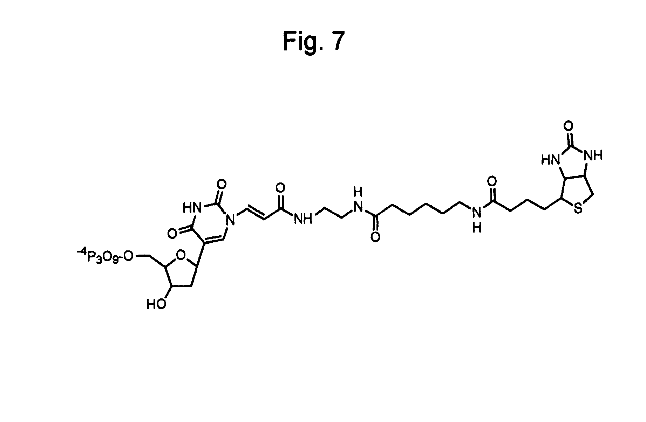

Figure 7 shows a 2'-deoxypseudouriding analog (i-DLR) which can be used for internal labeling of cDNA.

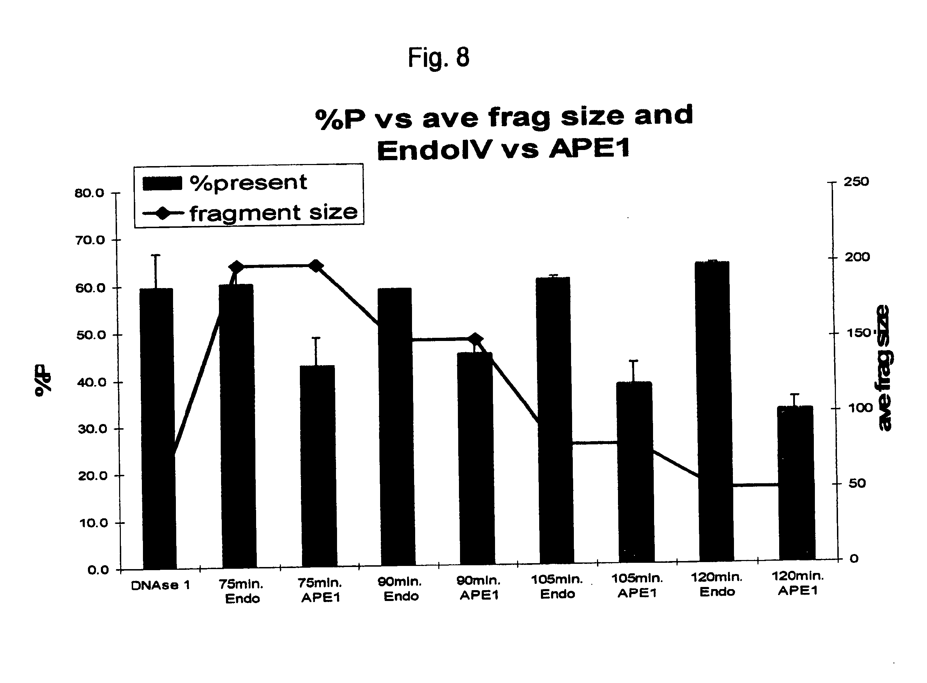

Figure 8 shows the hybridization results of Tris/Endo IV or APE 1 fragmentation and TdT labeling in percent present and also shows average fragment size.

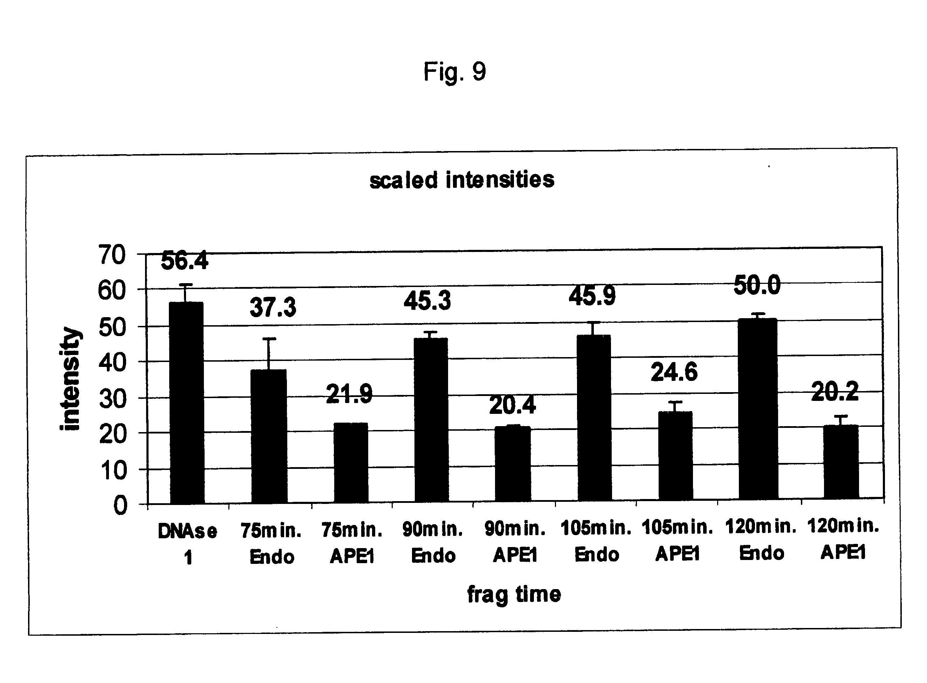

Figure 9 shows scaled intensity data for hybridization of samples fragmented with Tris/Endo IV or APE 1 labeled with DLR using TdT.

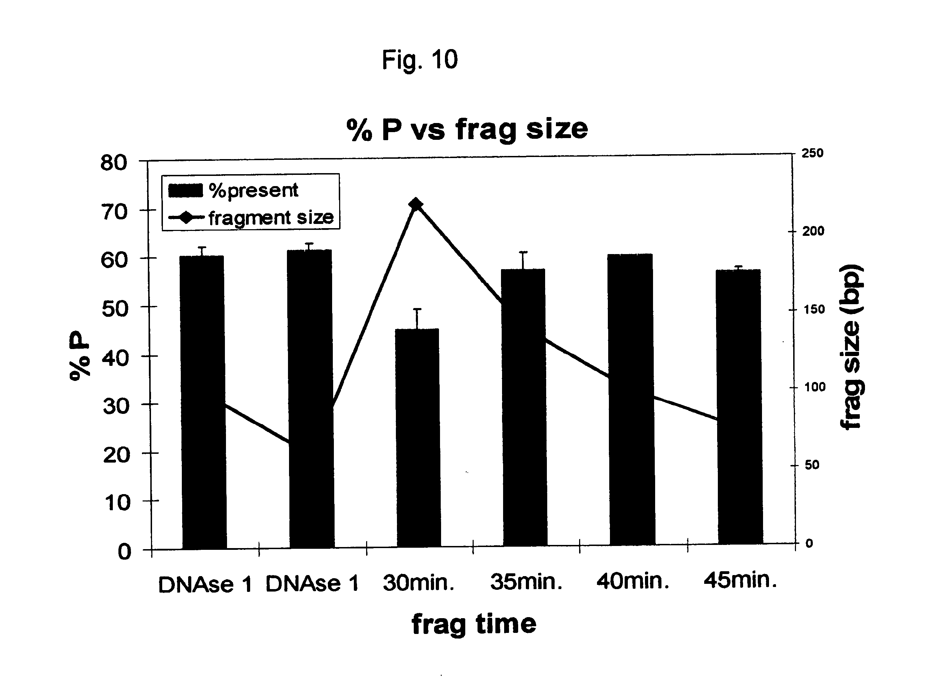

Figure 10 shows the hybridization results of fragmentation in 5 mM Tris with the addition of 5% NMF. Percent present and fragment size are shown compared to DNase I treated samples.

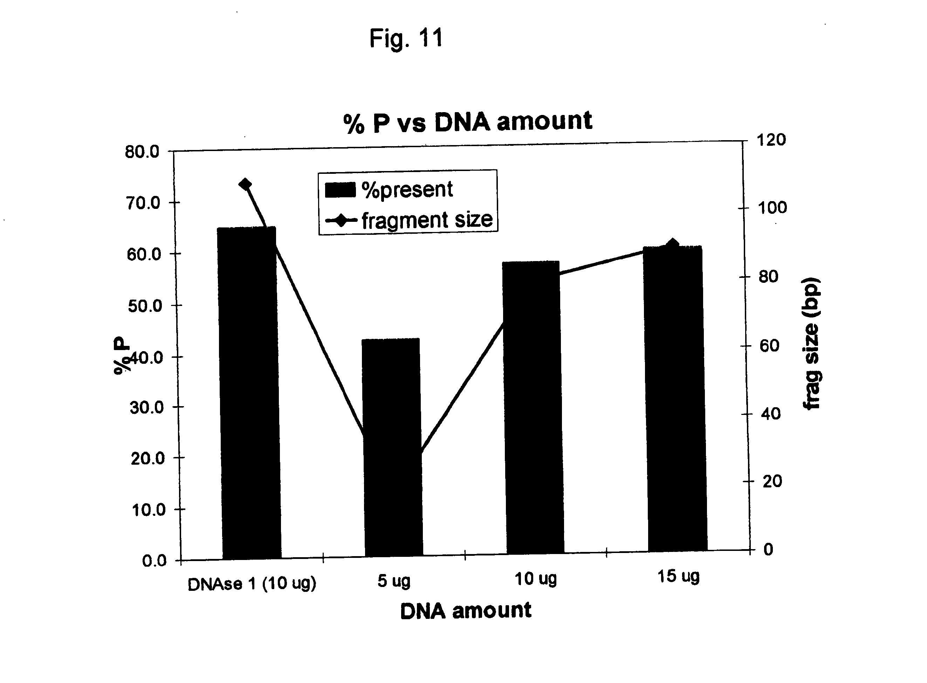

Figure 11 shows changes in percent present and fragmentation size in Tris plus NMF fragmentation in response to changes in DNA amount.

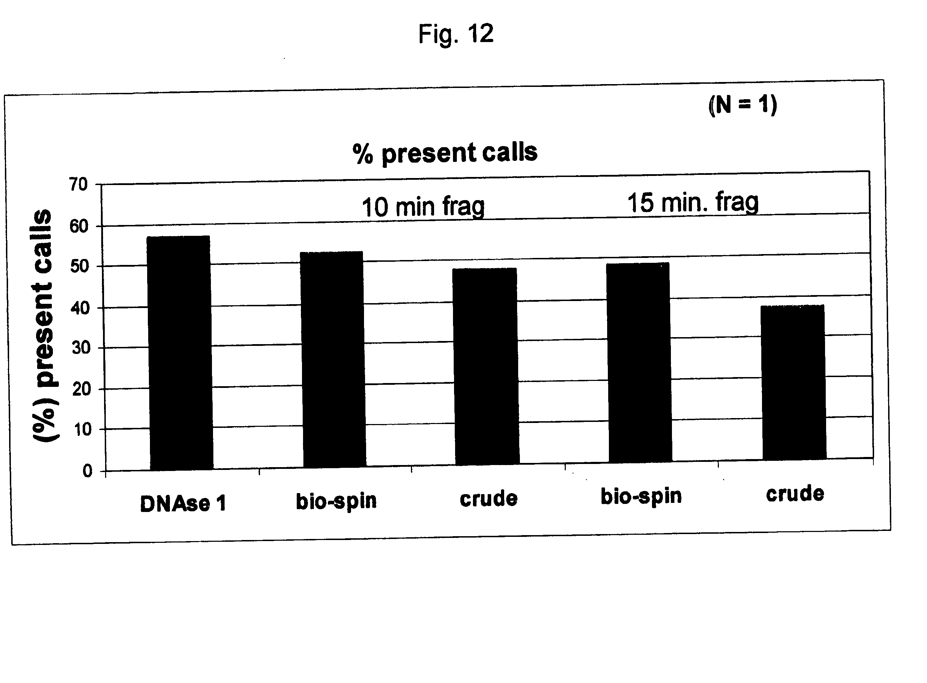

Figure 12 shows percent present calls after fragmentation of single stranded cDNA with Cu(OP)₂.
Figure 11 shows that the percent present and the fragment size vary depending on the amount of DNA present in the fragmentation reaction for Tris/Endo IV fragmentation with NMF present and TdT/DLR labeling.

### DETAILED DESCRIPTION OF THE INVENTION

The present invention has many preferred embodiments and relies on many patents, applications and other references for details known to those of the art.

As used in this application, the singular form "a," "an," and "the" include plural references unless the context clearly dictates otherwise. For example, the term "an agent" includes a plurality of agents, including mixtures thereof.

An individual is not limited to a human being but may also be other organisms including but not limited to mammals, plants, bacteria, or cells derived from any of the above.

Throughout this disclosure, various aspects of this invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The practice of the present invention may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques can be had by reference to the example herein below. However, other equivalent conventional procedures can, of course, also be used. Such conventional techniques and descriptions can be found in standard laboratory manuals such as Genome Analysis: A Laboratory Manual Series (Vols. I-IV), Using Antibodies: A Laboratory Manual, Cells: A Laboratory Manual, PCR Primer: A Laboratory Manual, and Molecular Cloning: A Laboratory Manual (all from Cold Spring Harbor Laboratory Press), Stryer, L. (1995) Biochemistry (4th Ed.) Freeman, New York, Gait, "Oligonucleotide Synthesis: A Practical Approach" 1984, IRL Press, Lond*on,* Nelson and Cox (2000), Lehninger, Principles of Biochemistry 3rd Ed., W. H. Freeman Pub., New York, NY and Berg et al. (2002) Biochemistry, 5th Ed., W.H. Freeman Pub., New York, NY.

The present invention can employ solid substrates, including arrays in some preferred embodiments. Methods and techniques applicable to polymer (including protein) array synthesis have been described in United States Serial No. 09/536,841, WO 00/58516, United States Patent Nos. 5,143,854, 5,242,974, 5,252,743, 5,324,633, 5,384,261, 5,405,783, 5,424,186, 5,451,683, 5,482,867, 5,491,074, 5,527,681, 5,550,215, 5,571,639, 5,578,832, 5,593,839, 5,599,695, 5,624,711, 5,631,734, 5,795,716, 5,831,070, 5,837,832, 5,856,101, 5,858,659, 5,936,324, 5,968,740, 5,974,164, 5,981,185, 5,981,956, 6,025,601, 6,033,860, 6,040,193, 6,090,555, 6,136,269, 6,269,846 and 6,428,752, in PCT Applications Nos. PCT/US99/00730 (International Publication Number WO 99/36760) and PCT/US01/04285.

Patents that describe synthesis techniques in specific embodiments include United States Patent Nos. 5,412,087, 6,147,205, 6,262,216, 6,310,189, 5,889,165, and 5,959,098. Nucleic acid arrays are described in many of the above patents, but the same techniques are applied to polypeptide arrays.

Nucleic acid arrays that are useful in the present invention include those that are commercially available from Affymetrix (Santa Clara, CA) under the brand name GeneChip®. Example arrays are shown on the website at affymetrix.com.

The present invention also contemplates many uses for polymers attached to solid substrates. These uses include gene expression monitoring, profiling, library screening, genotyping and diagnostics. Gene expression monitoring and profiling methods can be shown in United States Patents Nos. 5,800,992, 6,013,449, 6,020,135, 6,033,860, 6,040,138, 6,177,248 and 6,309,822. Genotyping and uses therefore are shown in USSN 60/319,253, 10/013,598, and United States Patent Nos. 5,856,092, 6,300,063, 5,858,659, 6,284,460, 6,361,947, 6,368,799 and 6,333,179. Other uses are embodied in United States Patents Nos. 5,871,928, 5,902,723, 6,045,996, 5,541,061, and 6,197,506.

The present invention also contemplates sample preparation methods in certain preferred embodiments. Prior to or concurrent with genotyping, the genomic sample may be amplified by a variety of mechanisms, some of which may employ PCR. See, *e.g.,* PCR Technology: Principles and Applications for DNA Amplification (Ed. H.A. Erlich, Freeman Press, NY, NY, 1992); PCR Protocols: A Guide to Methods and Applications (Eds. Innis, et al., Academic Press, San Diego, CA, 1990); Mattila et al., Nucleic Acids Res. 19, 4967 (1991); Eckert et al., PCR Methods and Applications 1, 17 (1991); PCR (Eds. McPherson et al., IRL Press, Oxford); and United States Patent Nos. 4,683,202, 4,683,195, 4,800,159, 4,965,188, and 5,333,675. The sample may be amplified on the array. See, for example, U.S. Patent No 6,300,070 and United States Patent Application 09/513,300.

Other suitable amplification methods include the ligase chain reaction (LCR) (e.g., Wu and Wallace, Genomics 4, 560 (1989), Landegren et al., Science 241, 1077 (1988) and Barringer et al. Gene 89:117 (1990)), transcription amplification (Kwoh et al., Proc. Natl. Acad. Sci. USA 86, 1173 (1989) and WO88/10315), self-sustained sequence replication (Guatelli et al., Proc. Nat. Acad. Sci. USA, 87, 1874 (1990) and WO90/06995), selective amplification of target polynucleotide sequences (United States Patent No. 6,410,276), consensus sequence primed polymerase chain reaction (CP-PCR) (United States Patent No. 4,437,975), arbitrarily primed polymerase chain reaction (AP-PCR) (United States Patent Nos. 5,413,909, 5,861,245) and nucleic acid based sequence amplification (NABSA). (See, United States Patents Nos. 5,409,818, 5,554,517, and 6,063,603). Other amplification methods that may be used are described in, United States Patent Nos. 5,242,794, 5,494,810, 4,988,617 and in United States Serial No. 09/854,317.

Additional methods of sample preparation and techniques for reducing the complexity of a nucleic sample are described in Dong et al., Genome Research 11, 1418 (2001), in United States Patent No 6,361,947, 6,391,592 and United States Patent Application Nos. 09/916,135, 09/920,491, 09/910,292, and 10/013,598.

Methods for conducting polynucleotide hybridization assays have been well developed in the art. Hybridization assay procedures and conditions will vary depending on the application and are selected in accordance with the general binding methods known including those referred to in: Maniatis et al. Molecular Cloning: A Laboratory Manual (2nd Ed. Cold Spring Harbor, N.Y, 1989); Berger and Kimmel Methods in Enzymology, Vol. 152, Guide to Molecular Cloning Techniques (Academic Press, Inc., San Diego, CA, 1987); Young and Davis, P.N.A.S, 80: 1194 (1983). Methods and apparatus for carrying out repeated and controlled hybridization reactions have been described in US patent 5,871,928, 5,874,219, 6,045,996 and 6,386,749, 6,391,623.

The present invention also contemplates signal detection of hybridization between ligands in certain preferred embodiments. See United States Patent Nos. 5,143,854, 5,578,832; 5,631,734; 5,834,758; 5,936,324; 5,981,956; 6,025,601; 6,141,096; 6,185,030; 6,201,639; 6,218,803; and 6,225,625, in United States Patent Application 60/364,731 and in PCT Application PCT/US99/06097 (published as WO99/47964).

Methods and apparatus for signal detection and processing of intensity data are disclosed in, for example, United Patent Nos. 5,143,854, 5,547,839, 5,578,832, 5,631,734, 5,800,992, 5,834,758; 5,856,092, 5,902,723, 5,936,324, 5,981,956, 6,025,601, 6,090,555, 6,141,096, 6,185,030, 6,201,639; 6,218,803; and 6,225,625, in United States Patent Application 60/364,731 and in PCT Application PCT/US99/06097 (published as WO99/47964).

The practice of the present invention may also employ conventional biology methods, software and systems. Computer software products of the invention typically include computer readable medium having computer-executable instructions for performing the logic steps of the method of the invention. Suitable computer readable medium include floppy disk, CD-ROM/DVD/DVD-ROM, hard-disk drive, flash memory, ROM/RAM, magnetic tapes and etc. The computer executable instructions may be written in a suitable computer language or combination of several languages. Basic computational biology methods are described in, e.g. Setubal and Meidanis et al., Introduction to Computational Biology Methods (PWS Publishing Company, Boston, 1997); Salzberg, Searles, Kasif, (Ed.), Computational Methods in Molecular Biology, (Elsevier, Amsterdam, 1998); Rashidi and Buehler, Bioinformatics Basics: Application in Biological Science and Medicine (CRC Press, London, 2000) and Oueletter and Bzevanis Bioinformatics: A Practical Guide for Analysis of Gene and Proteins (Wiley & Sons, Inc., 2nd ed., 2001). See United States Patent 6,420,108.

The present invention may also make use of various computer program products and software for a variety of purposes, such as probe design, management of data, analysis, and instrument operation. See, United States Patent Nos. 5,593,839, 5,795,716, 5,733,729, 5,974,164, 6,066,454, 6,090,555, 6,185,561, 6,188,783, 6,223,127, 6,229,911 and 6,308,170.

The present invention may also make use of the several embodiments of the array or arrays and the processing described in United States Patent Nos. 5,545,531 and 5,874,219.

Additionally, the present invention may have preferred embodiments that include methods for providing genetic information over networks such as the Internet as shown in United States Patent applications 10/063,559, 60/349,546, 60/376,003, 60/394,574, 60/403,381.

### b) Definitions

The term "array" as used herein refers to an intentionally created collection of molecules which can be prepared either synthetically or biosynthetically. The molecules in the array can be identical or different from each other. The array can assume a variety of formats, *for example,* libraries of soluble molecules; libraries of compounds tethered to resin beads, silica chips, or other solid supports.

The term "array plate" as used herein refers to a body having a plurality of arrays in which each microarray is separated by a physical barrier resistant to the passage of liquids and forming an area or space, referred to as a well, capable of containing liquids in contact with the probe array.

The term "combinatorial synthesis strategy" as used herein refers to a combinatorial synthesis strategy is an ordered strategy for parallel synthesis of diverse polymer sequences by sequential addition of reagents which may be represented by a reactant matrix and a switch matrix, the product of which is a product matrix. A reactant matrix is a 1 column by m row matrix of the building blocks to be added. The switch matrix is all or a subset of the binary numbers, preferably ordered, between 1 and m arranged in columns. A "binary strategy" is one in which at least two successive steps illuminate a portion, often half, of a region of interest on the substrate. In a binary synthesis strategy, all possible compounds which can be formed from an ordered set of reactants are formed. In most preferred embodiments, binary synthesis refers to a synthesis strategy which also factors a previous addition step. For example, a strategy in which a switch matrix for a masking strategy halves regions that were previously illuminated, illuminating about half of the previously illuminated region and protecting the remaining half (while also protecting about half of previously protected regions and illuminating about half of previously protected regions). It will be recognized that binary rounds may be interspersed with non-binary rounds and that only a portion of a substrate may be subjected to a binary scheme. A combinatorial "masking" strategy is a synthesis which uses light or other spatially selective deprotecting or activating agents to remove protecting groups from materials for addition of other materials such as amino acids.

The term "complementary" as used herein refers to the hybridization or base pairing between nucleotides or nucleic acids, such as, for instance, between the two strands of a double stranded DNA molecule or between an oligonucleotide primer and a primer binding site on a single stranded nucleic acid to be sequenced or amplified. Complementary nucleotides are, generally, A and T (or A and U), or C and G. Two single stranded RNA or DNA molecules are said to be complementary when the nucleotides of one strand, optimally aligned and compared and with appropriate nucleotide insertions or deletions, pair with at least about 80% of the nucleotides of the other strand, usually at least about 90% to 95%, and more preferably from about 98 to 100%. Alternatively, complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. See, M. Kanehisa Nucleic Acids Res. 12:203 (1984).

The term "genome" as used herein is all the genetic material in the chromosomes of an organism. DNA derived from the genetic material in the chromosomes of a particular organism is genomic DNA. A genomic library is a collection of clones made from a set of randomly generated overlapping DNA fragments representing the entire genome of an organism.

The term "hybridization" as used herein refers to the process in which two single-stranded polynucleotides bind non-covalently to form a stable double-stranded polynucleotide; triple-stranded hybridization is also theoretically possible. The resulting (usually) double-stranded polynucleotide is a "hybrid.". The proportion of the population of polynucleotides that forms stable hybrids is referred to herein as the "degree of hybridization". Hybridizations are usually performed under stringent conditions, for example, at a salt concentration of no more than 1 M and a temperature of at least 25°C. For example, conditions of 5X SSPE (750 mM NaCl, 50 mM NaPhosphate, 5 mM EDTA, pH 7.4) and a temperature of 25-30°C are suitable for allele-specific probe hybridizations. For stringent conditions, see, for example, Sambrook, Fritsche and Maniatis. "Molecular Cloning A laboratory Manual" 2nd Ed. Cold Spring Harbor Press (1989).

The term "label" as used herein refers to a luminescent label, a light scattering label or a radioactive label. Fluorescent labels include, *inter alia,* the commercially available fluorescein phosphoramidites such as Fluoreprime (Pharmacia), Fluoredite (Millipore) and FAM (ABI). See United States Patent 6,287,778.

The term "microtiter plates" as used herein refers to arrays of discrete wells that come in standard formats (96, 384 and 1536 wells) which are used for examination of the physical, chemical or biological characteristics of a quantity of samples in parallel.

The term "mixed population" or sometimes refer by "complex population" as used herein refers to any sample containing both desired and undesired nucleic acids. As a non-limited example, a complex population of nucleic acids may to total genomic DNA, total genomic RNA or a combination thereof. Moreover, a complex population of nucleic acids may have been enriched for a given population but include other undesirable populations. For example, a complex population of nucleic acids may be a sample which has been enriched for desired messenger RNA (mRNA) sequences but still includes some undesired ribosomal RNA sequences (rRNA).

The term "mRNA" or sometimes refer by "mRNA transcripts" as used herein, include, but not limited to pre-mRNA transcript(s), transcript processing intermediates, mature mRNA(s) ready for translation and transcripts of the gene or genes, or nucleic acids derived from the mRNA transcript(s). Transcript processing may include splicing, editing and degradation. As used herein, a nucleic acid derived from an mRNA transcript refers to a nucleic acid for whose synthesis the mRNA transcript or a subsequence thereof has ultimately served as a template. Thus, a cDNA reverse transcribed from an mRNA, an RNA transcribed from that cDNA, a DNA amplified from the cDNA, an RNA transcribed from the amplified DNA, *etc*., are all derived from the mRNA transcript and detection of such derived products is indicative of the presence and/or abundance of the original transcript in a sample. Thus, mRNA derived samples include, but are not limited to, mRNA transcripts of the gene or genes, cDNA reverse transcribed from the mRNA, cRNA transcribed from the cDNA, DNA amplified from the genes, RNA transcribed from amplified DNA, and the like.

The term "nucleic acids" as used herein may include any polymer or oligomer of pyrimidine and purine bases, preferably cytosine, thymine, and uracil, and adenine and guanine, respectively. *See* Albert L. Lehninger, PRINCIPLES OF BIOCHEMISTRY, at 793-800 (Worth Pub. 1982). Indeed, the present invention contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

The term "oligonucleotide" or sometimes refer by "polynucleotide" as used herein refers to a nucleic acid ranging from at least 2, preferable at least 8, and more preferably at least 20 nucleotides in length or a compound that specifically hybridizes to a polynucleotide. Polynucleotides of the present invention include sequences of deoxyribonucleic acid (DNA) or ribonucleic acid (RNA) which may be isolated from natural sources, recombinantly produced or artificially synthesized and mimetics thereof. A further example of a polynucleotide of the present invention may be peptide nucleic acid (PNA). The invention also encompasses situations in which there is a nontraditional base pairing such as Hoogsteen base pairing which has been identified in certain tRNA molecules and postulated to exist in a triple helix. "Polynucleotide" and "oligonucleotide" are used interchangeably in this application.

The term "primer" as used herein refers to a single-stranded oligonucleotide capable of acting as a point of initiation for template-directed DNA synthesis under suitable conditions for example, buffer and temperature, in the presence of four different nucleoside triphosphates and an agent for polymerization, such as, for example, DNA or RNA polymerase or reverse transcriptase. The length of the primer, in any given case, depends on, for example, the intended use of the primer, and generally ranges from 15 to 30 nucleotides. Short primer molecules generally require cooler temperatures to form sufficiently stable hybrid complexes with the template. A primer need not reflect the exact sequence of the template but must be sufficiently complementary to hybridize with such template. The primer site is the area of the template to which a primer hybridizes. The primer pair is a set of primers including a 5' upstream primer that hybridizes with the 5' end of the sequence to be amplified and a 3' downstream primer that hybridizes with the complement of the 3' end of the sequence to be amplified.

The term "probe" as used herein refers to a surface-immobilized molecule that can be recognized by a particular target. See U.S. Patent No. 6,582,908 for an example of arrays having all possible combinations of probes with 10, 12, and more bases. Examples of probes that can be investigated by this invention include, but are not restricted to, agonists and antagonists for cell membrane receptors, toxins and venoms, viral epitopes, hormones (for example, opioid peptides, steroids, etc.), hormone receptors, peptides, enzymes, enzyme substrates, cofactors, drugs, lectins, sugars, oligonucleotides, nucleic acids, oligosaccharides, proteins, and monoclonal antibodies.

The term "solid support", "support", and "substrate" as used herein are used interchangeably and refer to a material or group of materials having a rigid or semi-rigid surface or surfaces. In many embodiments, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations. See U.S. Patent No. 5,744,305 for exemplary substrates.

The term "target" as used herein refers to a molecule that has an affinity for a given probe. Targets may be naturally-occurring or man-made molecules. Also, they can be employed in their unaltered state or as aggregates with other species. Targets may be attached, covalently or noncovalently, to a binding member, either directly or via a specific binding substance. Examples of targets which can be employed by this invention include, but are not restricted to, antibodies, cell membrane receptors, monoclonal antibodies and antisera reactive with specific antigenic determinants (such as on viruses, cells or other materials), drugs, oligonucleotides, nucleic acids, peptides, cofactors, lectins, sugars, polysaccharides, cells, cellular membranes, and organelles. Targets are sometimes referred to in the art as anti-probes. As the term targets is used herein, no difference in meaning is intended. A "Probe Target Pair" is formed when two macromolecules have combined through molecular recognition to form a complex.

An abasic site or AP site in DNA or RNA results from loss of the base, frequently resulting from hydrolytic cleavage of the N-glycosylic bond. AP sites may also be oxidized, for example at the C-1', C-2', C-4' or C-5', resulting in modification of the deoxyribose moiety. The process is increased by any factor or chemical modification that develops a positive charge on the nucleic base and labilizes the glycosylic bond. Abasic sites are recognized by a set of endonucleases which recognize the AP site and cleave the DNA either at the 5' side of the AP site, *E.coli* exonuclease III and endonuclease IV, or at the 3' side of the AP site, for example, *E.coli* endonuclease III and bacteriophage T4 endonuclease V. may be cleaved by AP endonucleases. Abasic sites are also alkali-labile and can lead to strand breakage through β- and δ- elimination. For a discussion of abasic sites in DNA see Lhomme et al., Biopolymers 52-65-83 (1999). Generally all AP endonucleases recognize "regular" AP sites but may vary in their ability to recognize different oxidized AP sites, Povirk and Steighner Mutat. Res. 214:13-22 (1989) and Haring et al., Nuc. Acids Res. 22:2010-2015 (1994). AP endonucleases include, for example, FPG protein, endonuclease III, T4 endonuclease V, endonuclease IV and exonuclease III.

*E.coli* Endonuclease IV specifically catalyzes the formation of single strand breaks at apurinic and apyriminic sites in DNA. It also removes 3'-blocking groups (e.g. 3'-phosphoglycolate and 3'-phosphate) from damaged ends of DNA. Endonuclease IV is a class II AP (apurinic/apyrimidic) endonuclease with an associated 3'-diesterase activity and no associated N-glycosylase activity. Endonuclease IV can remove phosphoglycoaldhyde, deoxyribose-5-phospate, 4-hydroxy-2-pentanal, and phosphate groups from the 3' ends of DNA. Endonuclease IV does not contain 3' exonuclease activity. The enzyme has no magnesium requirement and is fully active in EDTA. The enzyme is further described in the following references: Ljungquist, S., et al., J. Biol. Chem., 252, 2808-2814 (1977), Levin, J.D., J. Biol. Chem., 263, 8066-8071 (1988), Demple, B. and Harrison, L., Annu. Rev. Biochem., 63: 915-948 (1994), and Levin, J.D. and Demple, B., Nucleic Acids Res., 24:885-889 (1996). APE 1 is described, for example, in Demple et al. P.N.A.S. 88:11450-11454 (1991).

Reference will now be made in detail to exemplary embodiments of the invention. While the invention will be described in conjunction with the exemplary embodiments, it will be understood that they are not intended to limit the invention to these embodiments. On the contrary, the invention is intended to cover alternatives, modifications and equivalents, which may be included within the scope of the invention.

### Chemical Fragmentation of Nucleic Acids for Array analysis

Microarray technology provides rapid, high-throughput, massively parallel methods for analysis of genetic information, including, for example, gene expression and genotype.
In many applications of the technology a sample containing nucleic acids to be analyzed is obtained and nucleic acids in the sample are amplified. Methods for amplification are well known in the art and include, for example, (1) amplification of the population of mRNA by reverse transcription using a primer that includes a polyT region and a promoter region for an RNA polymerase, such as T7, T3 or SP6, followed by in vitro transcription of many copies of the mRNAs from the starting material: (2) amplification of a representation of a genome by fragmenting the sample, ligating adaptors to the fragments and amplifying a subset of the fragments by PCR using a primer complementary to the adaptor sequence (whole genome sampling assay-WGSA) for additional description of WGSA see Matsuzaki et al., Gen. Res. 14:414-425 (2004); (3) other whole genome amplification methods such as multiple displacement amplification (MDA) and (4) the Whole Transcript Assays (WTA) which is described in greater detail below.

Methods for fragmentation and labeling nucleic acids for hybridization to nucleic acid arrays are disclosed. In preferred aspects the fragmentation method used is an alternative to methods that use DNaseI, such as those described in Wodicka et al., Nat. Biotech. 15: 1359-1367 (1997) and Matsuzaki et al., Gen. Res. 14:414-425 (2004). In many aspects DNA or RNA is amplified to generate an amplified DNA sample and the amplified sample is subjected to random fragmentation and labeling of fragments with a detectable label, such as biotin. The labeled fragments are hybridized to an array and the hybridization pattern may be detected and analyzed. In preferred aspects amplified samples are fragmented in preparation for labeling and hybridization to nucleic acid probe arrays. In one aspect the methods include a fragmentation step and a labeling step that may occur sequentially or simultaneously. The fragmentation step includes at least one chemical step, and the chemical step includes a treatment that generates abasic sites in the nucleic acid that may be cleaved to generate a strand break. In some aspects an AP endonuclease is used to cleave at abasic sites. In some aspects the fragmentation generates ends that are compatible with known methods of labeling nucleic acids, but in other aspects the fragments are subsequently treated to generate ends compatible with labeling. Some fragmentation methods may generate a mixture of ends and the mixture may be subsequently treated to generate ends compatible with labeling. In a particularly preferred embodiment the fragmentation and subsequent processing steps result in fragments that have a 3' OH and the fragments are substrates for end-labeling with terminal deoxynucleotidyl transferase (TdT).

In one aspect, fragmentation of nucleic acids comprises breaking nucleic acid molecules into smaller fragments. Fragmentation of nucleic acid may be desirable to optimize the size of nucleic acid molecules for subsequent analysis and minimize three dimensional structure. For example, fragmented nucleic acids allow more efficient hybridization of target DNA to nucleic acid probes than non-fragmented DNA and fragmented DNA that is to be end labeled allows for the incorporation of additional labels. According to a preferred embodiment, before hybridization to a microarray, target nucleic acid is fragmented to sizes ranging from about 40 to about 200 bases long, and more preferably from about 50 to about 150 bases long, to improve target specificity and sensitivity. In some aspects, the average size of fragments obtained is at least 10, 20, 30, 40, 50, 60, 70, 80, 100 or 200 bases. If the fragments are double stranded this length refers to base pairs and if single stranded this length refers to bases. Conditions of the fragmentation reaction may be optimized to select for fragments of a desired size range. One of skill in the art will recognize that a nucleic acid sample when fragmented will result in a distribution of fragment sizes, preferably the distribution is centered about a selected length, for example, the center of the distribution of fragment sizes may be about 20, 40, 50, 60, 70, 80 or 100 bases or base pairs. In a preferred aspect the methods reproducibly generate fragments that have approximately the same size distribution.

Chemical fragmentation methods that may be used include, for example, hydrolysis catalyzed by metal ion complexes, such as Cu⁺² and Ce⁺² complexes; oxidative cleavage by metal ion complexes, such as Fe⁺² and Cu⁺² complexes, photochemical cleavage, and acid-catalzyed depurination. The methods may also be used in conjunction with chemical DNA labeling methods, such as, biotin-amine, biotin-hydrazides, diazo-biotin, biotin-platinum, biotin-psoralen, and biotin-aryl azide methods.

In some aspects hydrolysis methods generate 5' phosphates and 3' hydroxyl ends which are compatible with labeling methods such as end labeling with terminal transferases and oxidative methods generate 5' and 3' carbonyl residues. Carbonyls may be chemically labeled, for example, with biotin-amines and -hydrazides. The phosphate backbone may be labeled, for example, with diazo-biotin and specific bases can be labeled, for example, with biotin-platinum, -psoralen and -aryl azide.

In preferred embodiments the methods may be used, for example, for fragmenting nucleic acid sample prior to labeling and hybridization to an array of probes. Preferred arrays of probes included high density arrays of oligonucleotides such as those made by Affymetrix, Inc. (Santa Clara, CA), for example, the 10K and 100K Mapping Arrays, tiling arrays, and expression arrays such as the Human Genome U133 Plus 2.0 array. The array may have probes for about 10, 20, 30, 40, 50, 75 or 100% of a selective genome. In one aspect the probes may be complementary to transcribed regions or to a combination of transcribed and non-transcribed regions. The array may include probes to detect each known or predicted exon in a plurality of genes, for example, more than 1,000, 2,000, 5,000, 10,000 or 30,000 genes.

In a preferred embodiment the nucleic acids to be fragmented by the disclosed methods are an amplification product. In one embodiment a biological sample containing RNA transcripts is amplified. The RNA may be used as template for a reverse transcription reaction to synthesize cDNA. Methods for synthesizing cDNA are well known in the art. Sample preparation for Whole Transcript Assays are described for example in U.S. Patent application Serial number 10/917,643. Enzymatic methods of fragmentation are also disclosed in U.S. Patent Application No. 10/951,983.

In another aspect the fragments are an amplification product resulting from Whole Genome Sampling Assay (WGSA) which is described, for example, in US patent publication Nos. 20040146890 and 20040067493. In general, genomic DNA is fragmented with one or more restriction enzymes, adaptors are ligated to the fragments and the adaptor ligated fragments are subjected to PCR amplification using a primer to the adaptor sequence. The PCR preferentially amplifies fragments that are less than about 2kb and greater than about 200 base pairs so a representative subset of the genome is amplified. The disclosed chemical fragmentation methods may be used to fragment the resulting WGSA amplification product prior to end labeling and hybridization to an array, for example, a genotyping array.

Both single-stranded and double-stranded DNA targets may be fragmented. The methods of the invention are particularly suitable for use with tiling array such as those described in U.S. Patent application Serial Number 10/815,333. While the methods of the invention have broad applications and are not limited to any particular detection methods, they are particularly suitable for detecting a large number of different target nucleic acids, such as more than 1000, 5000, 10,000, 50,000 different transcript features.

In a preferred aspect the fragments are end labeled using a terminal transferase enzyme (TdT). Terminal transferase catalyzes the template independent addition of deoxy- and dideoxynucleoside triphosphates to the 3'OH ends of double- and single-stranded DNA fragments and oligonucleotides. TdT can also add homopolymers of ribonucleotides to the 3' end of DNA. The preferred substrate for TdT is a protruding 3' end but the enzyme will also add nucleotides to blunt and 3'-recessed ends of DNA fragments. The enzyme uses cobalt as a cofactor. Terminal transferase may be used to incorporate, for example, digoxigenin-, biotin-, and fluorochrome-labeled deoxy- and dideoxynucleoside triphosphates as well as radioactive labeled deoxy- and dideoxynucleoside triphosphates. In a preferred embodiment a biotinylated compound is added by TdT to the 3' end of the DNA. In a preferred aspect fragments are labeled with biotinylated compounds such as those disclosed in US Patent Publication No. 20030180757. The biotin may be detected by contacting it with streptavidin with a fluorescent conjugate, such as Streptavidin-Phycoerythrin (Molecular Probes). A number of labeled and unlabelled streptavidin conjugates are available. Conjugates include fluorescent dyes such as fluorescein and rhodamine and phycobiliproteins such as phycoerythrin. Biotinylated antibodies to streptavidin may be used to amplify signal. For additional labeling methods, see for example, U.S. Patent Nos. 4,520,110 and 5,055,556. See also, U.S. Patent Application Nos. 10/452,519, which discloses labeling compounds and 10/617,992, which discloses labeling methods.

In some aspects the 3' end of fragments that are modified, for example, with a phosphoglycolate or 2' deoxyribolactone may be labeled using a 3' end repair system, tailing with dGTP/GTP and labeling with DLR using TdT. This is described in WO 03/050242. In some aspects, fragments may be labeled disproportionation and exchange of a labeled nucleotide to the 3' end by TdT in the presence of metal ions Co^{2+,} Mn²⁺ or Mg^{2+,} Co²⁺ being preferredl, as described in Anderson et al., Nuc. Acids Res. 27:3190-3196 (1999). Optimal concentration of the metal ion is 1-2 mM.

There are a number of chemical methods for fragmentation of nucleic acids that are known in the art. These methods include: hydrolytic methods (see Sreedhara et al., J. Amer. Chem Soc. 2000, 122, 8814-8824), oxidative-based metallo-nucleases (see Pogozelski and Tullius, Chem. Rev. 1998, 98:1089-1107 and James G. Muller; et al., Chem. Rev. 1998, 98:1109-1151), photocleavage (see Nielson, J. Amer. Chem. Soc., 1992, 114:4967-4975), acid catalyzed depurination, (see Proudnikov and Mirzabekov, Nucleic Acids Res. 1996, 24, 4535-4532), alkylation (see Kenneth A. Browne, Amer. Chem. Soc. 2002, 124, 7950-7962) or fragmentation facilitated by reagents used in Maxam-Gilbert type sequencing methods. Fragmentation of DNA in low salt buffers at pH 6-9 has also been reported, see, for example, WO 03/050242 A2, US 20030143599 and US 20040209299.

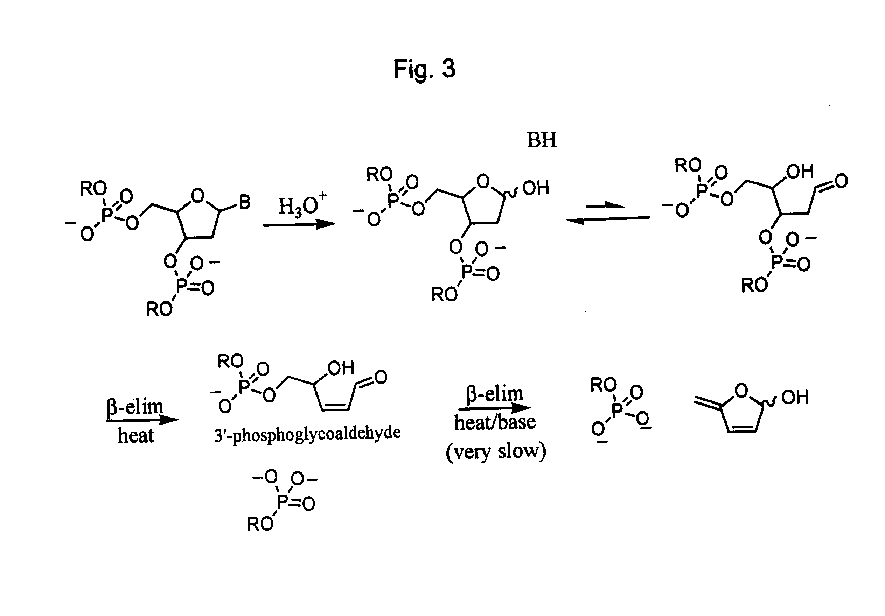

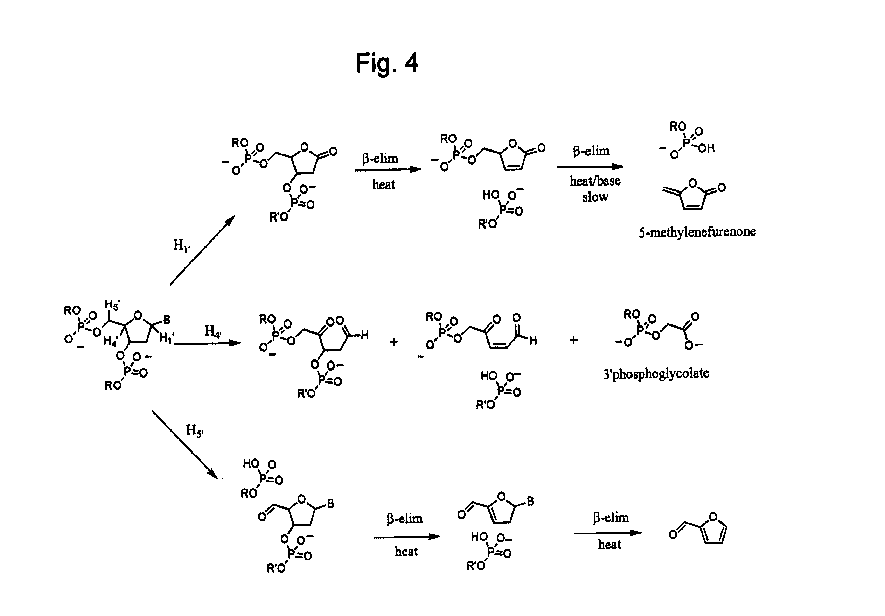

In preferred embodiments amplified DNA is incubated under conditions that result in acid catalyzed depurination as shown in Figure 3. The reaction can generate a mixture of products. In the first step an abasic site is generated. The depurination does not break the phosphate backbone but depurinated positions are reactive and can result in strand breakage as shown, generating a variety of 5' and 3' ends for the resulting fragments. The abasic product can undergo beta elimination resulting in fragmentation and generating a 3' phosphoglycoaldehyde and a 5' phosphate product as shown. A second beta elimination can also take place generating a 3' phosphate end. The second beta elimination occurs slowly but can be facilitated by addition of base, for example NaOH. The 3'-phosphoglycoaldehyde can be labeled chemically, for example, by biotin-ARP.

The DNA may be single stranded or double stranded and may be cDNA derived from mRNA or amplified products from a sample containing genomic DNA. In a preferred embodiment the DNA is in a solution that includes a buffer that is neutral (pH 6 to 9) at a temperature of about 22-30°C but acidic (pH less than 6.0) at higher temperatures, for example between 80 and 100°C. In a preferred aspect the DNA is in a solution that includes about 10 mM Tris-HCl, pH ~7.2-7.5 at 25°C. The pH of Tris buffer changes at a rate of-0.028 pH units per degree so if the pH is ~7.2-7.5 at about 25°C it will be ~5.2-5.5 at about 95°C, resulting in an acidic environment at high temperature and facilitating depurination of the DNA and generates abasic sites in the DNA at the site of depurination. For additional description of Tris buffers see Bates and Bower, Analyt. Chem. 28:1322 (1956) and Bates and Hetzer, Analyt. Chem. 33:1285 (1960).

Abasic sites can be treated by a variety of methods to generate strand breaks thus generating free 3' and 5' ends that can be labeled. In preferred embodiments the depurination reaction is incubated for about 10 to 30 or about 30 to 60 minutes. The fragments produced may be treated with Endonuclease IV or other 3'-end conditioning enzymes (like APE 1) to facilitate removal of 3'-modifications, such as 3' phosphates, facilitating efficient labeling with TdT.

In some aspects the mechanism of fragmentation is acid catalyzed depurination followed by thermal fragmentation or fragmentation by an AP endonuclease or a combination of treatments. Following acid depurination with thermal fragmentation generally results in incomplete fragmentation and generates fragments with 3' modifications, like those previously described by Proudnikov; et al., Nucleic Acids Research 1996, 24, 4535-4532, that may be compatible with chemical labeling methods, for example, labeling with biotin-amine, but are generally not compatible with TdT labeling, In preferred aspects the sample is treated with an AP endonuclease to cleave at unfragmented abasic sites and to remove 3' modifications, leaving 3'-hydroxyl groups that are compatible with TdT labeling. In a preferred embodiment *E.coli* Endo IV or the human Endo IV homolog, APE 1, is used after acid depurination, with or without heat treatment, to generate strand breaks at residual abasic sites and to remove 3' end blocking groups, leaving free 3'-hydroxyls that can be efficiently end-labeled by TdT.

DNA is mixed with a buffer that is neutral or basic in a first temperature range and acidic in a second temperature range. The DNA is mixed with the buffer at a temperature within the first temperature range and then incubated at a temperature in the second temperature range. The buffer is acidic in the second temperature range and the DNA is fragmented or apurinic sites are generated that may be subsequently fragmented by chemical or enzymatic means. Depurination and fragmentation may be stopped by returning the reaction to a temperature in the first temperature range, where the pH of the buffer is neutral. After fragmentation the sample may be treated with a nuclease such as Endo IV or APE1 prior to labeling. Fragmentation reactions that result in generation of 3' phosphates may be followed by treatment with a phosphatase to remove the phosphate and generate 3' hydroxyl for labeling, Endo IV also has a 3' phosphatase activity.

Many buffers are available that are neutral or basic at a first temperature range and acidic at a second temperature range. For detailed information about buffers see, for example, Data for Biochemical Research, 3rd Edition, Eds. Dawson et al. Oxford Scientific Publications (1995), see especially pages 417-448. In a preferred embodiment the buffer is Tris-HCl (other counter ions may also be used). Other buffers that change from a neutral pH at about 20 to 30°C to an acidic pH at about 85-100°C may also be used. Other buffers that may be used include, for example, TE, imidazole and colamine (2-aminoethanol/ethanolamine/2-hydroxyelylamine). Fragmentation can be stopped by changing the incubation temperature back to a temperature that results in a neutral or basic pH. This is particularly useful for high throughput sample preparation methods because the reaction can be stopped by changing the temperature so it can be done rapidly and without the need to add reagents. Incubation at the higher temperature may be for 10-30 min, 25-30 min, 30-40 min, 40-50 min, 50-60 min or 60-120 min or longer. In a preferred embodiment the incubation is for about 10, 20, 30, 40, 45 or 60 minutes. The fragmentation reaction may then be incubated in TdT buffer with 70 units Endo IV for 37°C for about 2 hours then at 70°C for 15 minutes. End labeling may be with TdT and Affymetrix biotinylated DNA Labeling Reagent (DLR). See also, U.S. Patent Application Nos. 60/545,417, 60/542,933, 60/512,569, 10/452,519 and 10/617,992.

In one embodiment 3 µg single stranded cDNA in 10 mM TE, pH 7.4 is incubated at 95°C for 30, 40, 45 or 60 minutes. TdT buffer and 70 units Endo IV is added and incubated at 37°C for 2 hours then at 70°C for 15 minutes. The reaction is then end labeled with Affymetrix biotinylated DNA Labeling Reagent, DLR (Affymetrix, Santa Clara, CA, USA) using TdT and hybridized to an array under standard conditions. Fragment sizes were about 80 base pairs after a 45 min incubation and about 50 base pairs after a 60 minute incubation. These fragment sizes are similar to what is observed with DNase I treatment and hybridization results were also similar. In another example fragmentation was with 1X TE pH 7.4 for 30 or 40 min at 95°C and 100 U of APE 1 or 70 U of Endo IV were used. In another embodiment 10 mM Tris-HCl buffer, pH 7.2 is used for fragmentation. Fragmentation rates for double stranded cDNA may be slower than single stranded cDNA.

See also, U.S. Patent Application Nos. 60/545,417, which discloses methods of fragmentation, 60/542,933, which discloses methods of whole transcript amplification, 10/452,519, which discloses labeling compounds and 10/617,992, which discloses labeling methods. In preferred embodiments the resulting fragments range in size from about 50 to about 200 base pairs, and more preferably from about 50 to about 100.

In a preferred embodiment the multiple copies of cDNA generated by the disclosed methods are analyzed by hybridization to an array of probes. The nucleic acids generated by the methods may be analyzed by hybridization to nucleic acid arrays. Those of skill in the art will appreciate that an enormous number of array designs are suitable for the practice of this invention. High density arrays may be used for a variety of applications, including, for example, gene expression analysis, genotyping and variant detection. Array based methods for monitoring gene expression are disclosed and discussed in detail in U.S. Pat. Nos. 5,800,992, 5,871,928, 5,925,525, 6,040,138 and PCT Application WO92/10588 (published on Jun. 25, 1992). Suitable arrays are available, for example, from Affymetrix, Inc. (Santa Clara, CA). Bead based array systems may also be used.

In another aspect N-methylformamide (NMF) is included in the fragmentation reaction. The Maxam-Gilbert type fragmentation chemistry in one approach uses a concentrated aqueous solution (~80%) of formamide which reacts with purines and pyrimidines at high temperature (>100°C) resulting in deglycosylation, see Raffaele Saladino; et al., J. Amer. Chem. Soc. 1996, 118, 5615-5619. Subsequent heating and base treatment, for example with piperidine, may be used to facilitate the β-elimination and fragmentation reactions to produce 5' and 3'-phosphate modified DNA fragments. In another modification of this procedure, it was discovered that NMF in the presence of 3 mM MnCl₂ at 110°C could effect both deglycosylation and fragmentation simultaneously, see Rodolfo Negri; et al. BioTechniques, 21, 910-917 (1996). This reaction, although sufficient for sequencing protocols, is relatively inefficient and may not result in complete fragmentation.

In one aspect of the present invention methods for fragmenting in the presence of NMF are disclosed. The methods preferably generate fragments with 3' hydroxyl groups that are substrates for labeling by TdT. In some aspects NMF is added to the fragmentation reaction to increase the rate of fragmentation. In a preferred embodiment a reagent formulation of between 5 and 10% NMF in tris or phosphate buffer at about pH 7 to 8.5 at 95°C is used. In a preferred embodiment the fragmentation proceeds for 30 to 60 minutes.
In some embodiments the single stranded DNA may be fragmented for less time than double stranded, for example, about 30 min for ssDNA and about 60 min for dsDNA. Double and single-stranded DNA may be fragmented by the disclosed methods and may be desalted prior to fragmentation.

The resulting fragments may be treated with an endonuclease, such as Endo IV, or other 3'-end conditioning enzyme, for example, APE 1, to facilitate deglycosylation and to remove 3'-modifications. Endo IV treatment may be by addition of TdT buffer, CoCl2 and Endo IV followed by incubation at 37°C for about 1, 2 or 3 hours and then at 65°C for 5-30 min, preferably about 15 min. For APE1 treatment NEB buffer and APE1 may be added to the fragmentation reaction and incubation may be for 1-3 hours at about 37°C, followed by incubation at 95°C for about 5 min.

The fragments may then be end labeled with a detectable label, for example, by TdT end labeling. End labeling of the Endo IV reaction mixture may be by addition of DNA labeling reagent (DLR) and TdT followed by incubation at 37°C for about 1 hour followed by addition of EDTA. For the APE1 treated sample labeling may be by the addition of TdT buffer, CoCl₂, DLR and TdT, followed by incubation at 37°C for about 1 hour. The reaction may be stopped by addition of EDTA. The labeled fragments may then be hybridized to an array of nucleic acids, for example oligonucleotide or cDNA arrays. The resulting hybridization pattern may be analyzed to measure the presence or absence of targets and to approximate the amount of individual targets in the starting sample.

DNA may also be fragmented using metal complexes as catalysts for oxidative fragmentation of DNA. In general metallo-based oxidative methods for DNA cleavage use a metal complex in the presence of an oxidant like oxygen or hydrogen peroxide and may use a reductant which at elevated temperature results in oxidation of the sugar backbone. Subsequent heating or base treatment, for example, treatment with piperidine, may be used to facilitate the beta-elimination and fragmentation reactions to generate 5' and 3' phosphate modified DNA fragments.

In preferred embodiments the fragments may be treated with an AP endonuclease, such as Endonuclease IV or another 3' end conditioning enzyme, such as APE 1 to facilitate deglycosylation and removal of 3' modifications to facilitate efficient end labeling, for example, with TdT.

Known chemical nucleases that nick nucleases under physiological conditions include the 1,10-phenanthroline-copper complex, derivatives of ferrous-EDTA, various metalloporphoryins and octahedral complexes of 4,7-diphenyl-1,10-phenanthroline. Bis (1,10-phenanthroline)copper (II) (abbreviated Cu(OP)₂) degrades DNA in the presence of coreactants, such as hydrogen peroxide and ascorbate. For more information on cleavage by Cu(OP)₂ see Pogozelski and Tullius (1998) at pp 1094-1095 and Signam, Biochemistry 29:9097-9105 (1990). In one mechanism proposed for DNA cleavage by Cu(OP)₂ strand breakage is observed at room temperature and does not require heat and alkali treatment.

Metal complexes such as Cu(OP)₂ and Fe⁺² (EDTA) in the presence of hydrogen peroxide can be used to fragment cDNA efficiently and reproducibly. Treatment of DNA or RNA results in abstraction of a hydrogen from the sugar moiety, producing a carbon-based radical that can rearrange to generate a reactive abasic site as a result of deglycosylation. The abasic site can be subsequently cleaved to generate a strand break. Cleavage at the abasic site may be by a variety of mechanisms that may be chemical or enzymatic. In a preferred aspect, for example, by an AP endonuclease. The fragments can be labeled with DLR by TdT with an efficiency greater than or equal to 95%. The fragments can be hybridized to probe arrays. The DNA may be incubated with a concentration of Cu(OP)₂ between about 0.75 mM to about 1.5 mM. The DNA may be incubated at 95°C to further fragment abasic sites. Endo IV or APE1 may be used to give 3'-OH ends.

A protocol and reagent formulation containing a copper-phenanthroline complex (Cu(OP)₂) and a reductant are enclosed.

A reagent formulation of about 5 µM Cu(OP)₂ with about 1 mM sodium ascorbate (C₆H₇O₆Na) or 10 mM mercaptopropionic acid (HSCH₂CH₂COOH) in a tris or phosphate buffer pH 7-8.5 at 65°C may be used to fragment single and double stranded DNA. The fragmentation reaction may proceed for about 10 to 30, or about 30 to 60 minutes at about 65°C.

Iron-EDTA complex (Fe⁺²(EDTA)) in the presence of hydrogen peroxide may be used for fragmentation. In the Fenton-Udenfriend reaction [Fe(EDTA)]²⁻ is oxidized by hydrogen peroxide generating highly reactive hydroxyl radicals. The Fenton-generated hydroxyl radical is diffusible and can cleave nucleic acids without specificity for a particular nucleotide. The hydroxyl radical is able to abstract hydrogen from each deoxyribose carbon but the 5' and 4' positions are preferred.

Copper derivatives of aminoglycosides have been shown to be highly efficient catalysts for cleavage of DNA under physiological conditions. See Sreedhara et al., J.Am.Chem.Soc., 122: 8814-8824, (2000), and Sreedhara et al., Chem. Commun., 1147 (1999). Strand cleavage at the abasic sites may be by heating the reaction mixture, for example at 85°C for about 20 min or by an AP endonuclease, for example, Endo IV and APE 1. The copper aminoglycoside, copper neamine, may also result in nucleic acid cleavage in the presence of peroxide or ascorbate. See, Patwardhan and Cowan, Chem. Commun., 1490-1491 (2001).

A copper kanamycin Complex (Cu(kanA) or Cu(kanA)₂) may be used for hydrolytic cleavage of DNA. Chemical fragmentation of nucleic acid may be by way of a hydrolytic mechanism resulting in phosphodiester hydrolysis. Examples of reagents that may be used to catalyze hydrolysis include transition metals and lanthanides, such as Cu(kanA), Ce(EDTA) and Ce₂(HXTA). Generally these reagents fragment by a hydrolytic mechanisms that is generally slower than DNase-1 and generates 5' phosphate and 3' hydroxyl end that are compatible with TdT labeling and chemical labeling. In one aspect a dicerium complex, Ce₂(HXTA) may be used for cleavage of nucleic acid. (HXTA = 5-methyl-2-hydroxy-1,4-xylene-alpha, alpha-diamine-N,N,N',N'-tetraacetic acid.) Ce(2)(HXTA) has been shown to hydrolyze DNA at pH 8 and 37°C. See, Branum et al. J. Am. Chem. Soc. 123:1898-904 (2001). A large percentage of the fragments, more than 90%, have 3'-OH ends, ready for end labeling, for example, by TdT.

Examples of reagents that cleave via an oxidative sugar fragmentation include, for example, fenton-type reagents such as Fe(EDTA)/H₂O₂, Cu(phen)/H₂O₂ and metalloporphyrin complexes and photochemical reagents such as Rh⁺³ complexes and uranyl acetate. The mechanism of cleavage is oxidative, the rate of cleavage is comparable to DNase-1 and results in fragments that have 3'-modifications. Acids, such as formic acid, can be used to fragment via a depurination method. The rate of cleavage is comparable to DNase I, and fragments with 3' modifications are generated.

In another aspect DNA may be cleaved by a first step involving acid catalyzed depurination followed by cleavage with a beta-lyase. Examples of β-lyases that may be used include, *E. coli* endonuclease III, T4 endonuclease V and *E. coli* FPG protein. Many β -lyases generate a strand break at the 3' side of the AP site by a β-elimination mechanism, see Mazumder et al., Biochemistry 30:1119 (1991). An exemplary schematic is shown in Fig. 6. In a first step the DNA (1) is depurinated. Depurination may be, for example, by incubation in a buffer that has a pH of about 5 at 95°C, for example Tris. The depurinated DNA is then cleaved using a beta-lyase, for example, Endo III. In a preferred aspect a thermostable beta-lyase that is functional at pH below 6 may be used so that depurination and cleavage can occur in the same reaction, simultaneously. A thermostable endonuclease II homolog is available, see Yang et al., Nuc. Acids Res. 29:604-613 (2001). The cleavage generates 5' phosphate ends and 3' phosphoglycoaldehyde ends, as shown (4). The fragments can be end labeled with a biotin amine reagent, for example, biotin-ARP (biotin aldehyde-reactive probe) (Molecular Probes), resulting in imine (5). Labeling may also be performed using reductive amination with RNH₂, for example incubation with Biotin-NH₂ and NaBH₄ or NaCNBH₃, may be used to generate a stable amine (6), see Kelly et al., Analytical Biochem. 311:103-118 (2002) and Fig 6. The biotin-ARP (or ARP-biotin) is a biotinylated hydroxylamine that reacts with aldehyde groups formed when reactive oxygen species depurinate DNA. The reaction forms a covalent bond linking the DNA to biotin. The biotin can then be deteced using a fluorophore- or enzyme-linked streptavidin.

A labeled nucleotide such as the one shown in Fig. 7 may be incorporated into the first strand cDNA during reverse transcription. The strand with the incorporated label can be fragmented using DNase I, Cu(OP)₂ or the Tris methods described above. Incorporation of a label during synthesis eliminates the need to label the fragments after fragmentation by, for example, TdT labeling or chemical labeling of the fragments.

### EXAMPLES

### Example 1: Fragmentation of single-stranded DNA in Tris Buffer at high temperature.

Fragmentation Reaction Mix: Mix 3 µl 10 X Tris Buffer, pH 7.24 at room temp, 20-25 µl ss cDNA (final concentration is 3 µg), and nuclease free water to a total volume of 30 µl. Incubate the reaction at 95°C for 60 minutes. The fragmented cDNA is applied directly to Endo IV treatment and the terminal labeling reaction. Alternatively, the material can be stored at -20°C for later use.

Endo IV treatment: Mix14 µl 5 X TdT Reaction Buffer (final concentration is 1X), 14 µl 25 mM CoCl₂ (final concentration is 5mM), 3.5 µl Endo IV (20 U/µl) (final concentration is 70U/3µg cDNA), 30 µl cDNA template (1.5-5 µg) and Nuclease-free H₂O for a final volume of 70 µl. Higher concentrations of Endo IV have been observed to result in more efficient labeling. Incubate the reaction at 37°C for 120 minutes. Inactive Endo IV at 65°C for 15 minutes.

Terminal Label Reaction: Mix 70 µl cDNA template (1.5-5 µg), 4.375 µl rTDT (400 U/ul) for final concentration of 5.8 U/pmol, and µl 5 mM DLR for final concentration of 0.07 mM. The final reaction volume is about 75.4 µl. Incubate the reaction at 37°C for 60 minutes. Stop the reaction by adding 2 µL of 0.5 M EDTA (PH 8.0). The target is ready to be hybridized onto probe arrays. Alternatively, it may be stored at -20°C for later use.

### Example 2: Fragmentation of ds cDNA with Tris Buffer at high temperature.

Fragmentation mixtures containing 10 µg ds cDNA, 10 mM Tris-HCl, pH 7.2 at room temperature were incubated at 95°C for 75, 90, 105 and 120 minutes. The reactions were then treated by either: (A) incubation with 100 unites APE 1, in NEB buffer 4 for 1 hour at 37°C and then 95°C for 50 min or (B) incubation with 70 units Endo IV in TdT buffer for 2 hours at 37°C and 15 min at 65°C. Both were then end labeled with DLR and TdT and hybridized to arrays using standard conditions. For those reactions that were treated with APE 1 the average size of fragments was approximately 200, 150, 90 or 60 bp after 75, 90, 105 or 120 min of incubation, respectively. For those reactions that were treated with Endo IV the average size of fragments was approximately 160, 110, 80 or 50 bp after 75, 90, 105 or 120 min of incubation, respectively. Percent present calls were 60.2, 58.9, 60.7, and 63.4 for Endo IV treated samples at 75, 90, 105 and 120 min respectively and 42.7, 45.0, 38.1, and 32.1 for APE 1 treated samples at 75, 90, 105 and 120 min respectively.

Results are shown in Fig. 8 as percent present (%P) and average fragment size compared to a DNase I control. Scaled intensity data is shown in Fig. 9.

### Example 3: NMF fragmentation with 10 or 20% NMF.

Fragmentation was tested at 10% NMF for 60 min or 20% NMF for 30 min, both at 100°C using cDNA in ~10 mM Tris-HCl buffer at pH 8 at 25°C. The NMF did not interfere with the activities of Endo IV or TdT enzymes.

Tubes 1-6 were incubated at 100°C for 90 min and tubes 7-12, w1 and w2 were incubated at 100°C for 40 min. Reactions were as indicated in Table 1.

**Table 1.**

| Reaction | Water (µl) | cDNA | Buffer | CoCl | Enzyme | SAP | Total volume | NMF |
|---|---|---|---|---|---|---|---|---|
| 1 | 23 | 15µl | 14µl 5x | 14µl | 6µl EndoIV | -- | 72µl | 10% |
| 2 | 23 | 15µl | 14µl 5x | 14µl | 6µl EndoIV | -- | 72µl | 10% |
| 3 | 17 | 15µl | 14µl 5x | 14µl | -- | 12 µl | 72µl | 10% |
| 4 | 17 | 15µl | 14µl 5x | 14µl | -- | 12 µl | 72µl | 10% |
| 5 | 20 | 15µl | 5µl 10x NEB | -- | 10µl APE | -- | 50µl | 10% |
| 6 | 20 | 15µl | 5µl 10x NEB | -- | 10µl APE | -- | 50µl | 10% |
| 7 | 23 | 15µl | 14µl 5x | 14µl | 6µl | -- | 72µl | 20% |
| 8 | 23 | 15µl | 14µl 5x | 14µl | 6µl EndoIV | -- | 72µl | 20% |
| 9 | 17 | 15µl | 14µl 5x | 14µl | -- | 12 µl | 72µl | 20% |
| 10 | 17 | 15µl | 14µl 5x | 14µl | -- | 12 µl | 72µl | 20% |
| 11 | 20 | 15µl | 5µl 10x NEB | -- | 10µl | -- | 50µl | 20% |
| 12 | 20 | 15µl | 5µl 10x NEB | -- | 10µl APE | -- | 50µl | 20% |
| W1 | 29.3 | 10µl | 4.5µl 10x one phor-all | -- | 1.2µl DNase I | -- | 45µl | -- |
| W2 | 29.3 | 10µl | 4.5µl 10x one phor-all | -- | 1.2µl DNase I | -- | 45µl | -- |

After fragmentation the products were end labeled using DLR and TdT. For labeling 1 µl of DLR and 4.4 µl of TdT were added to tubes 1-4 and 7-10 and 14 µl 5x buffer, 14 µl of CoCl₂, 1 µl of DLR and 4.4 µl of TdT were added to tubes 5, 6, 11, 12, w1 and w2. After hybridization to a test array the percent present were as follows: 59.8% for w1 and w2 controls, 48.7% for 10% NMF Endo IV, 36.3% for 10% NMF SAP, 39.6% for 10% NMF APE, 39.7% for 20% NMF Endo IV, 18.3% for 20% NMF SAP and 30.1% for 20% NMF APE. Background measurements were similar for all conditions.

### Example 4: Fragmentation in a reaction including 5% NMF.

1.5µl of 50% aqueous NMF is added to 10 µl of ~ 3µg DNA in 1mM Tris or phosphate buffer, followed by 3.5 µl of H₂O to a final reaction volume of 15µl. The fragmentation mixture is incubated at 95°C about 30 min for ss-DNA and about 60 min. for ds-DNA.

Deglycosylation and removal of 3'-modifications: Endo IV treatment: 14 µl of 5xTdT buffer, 14µl of 25mM CoCl₂ and 6µl of Endo IV (2U/µl) is added to the 15 µl of fragmentation mixture. (Higher concentrations of Endo IV may be used, for example, instead of 12 units about 70 units or more may be used.) Add water to make the final reaction volume 70µl. Incubate at 37°C for 2 hours and at 65°C for 15 min. 3'-end labeling with TdT and DLR reagent: Endo IV reaction mixture: 1 µl of DNA labeling reagent and 4.4 µl of TdT (400U/µl) is added to 70µl of reaction mixture and incubated at 37°C for 1 hour, followed by the addition of 2µl of 0.5M EDTA, pH 8.

APE 1 may be used instead of EndoIV as follows: 5 µl 10x NEB buffer and 10µl of APE 1 (10U/µl) is added to 15 µl of fragmentation mixture. Add water to a final reaction volume of 50µl. Incubate at 37°C for 2 hours and at 95°C for 5 min.

3'-end labeling with TdT and DLR reagent: APE 1: add 14 µl of 5xTdT buffer, 14µl of 25mM CoCl₂, 1 µl of DNA labeling reagent and 4.4 µl of TdT (400U/µl) to 50 µl of reaction mixture. Incubate at 37°C for 1 hour followed by the addition of 2µl of 0.5M EDTA, pH 8. Hybridize labeled fragments to an array according to standard protocols.

Results for Tris fragmentation in the presence of 5% NMF are shown in Fig. 10. The percent present observed is comparable to DNase I. The observed rate of fragmentation in the presence of 5% NMF was about two-fold faster than in the absence of NMF. This was observed for both single and double-stranded cDNA. The observed scaled signal intensities were 26.7 at 30 min, 27.8 at 35 min, 26.9 at 40 min and 28.5 at 45 min, compared to 47.9 and 41.9 for DNase I at 1/100 bp and 1/60 bp respectively.

### Example 5: Tris/Endo IV fragmentation with 5 or 10% NMF.

Desalted plasmid DNA was fragmented in 5 or 10mM Tris-HCL buffer, pH7.2 with 0, 5 or 10% NMF and desalted double stranded cDNA was fragmented in 5 mM Tris-HCl buffer with or without 5% NMF. Fragmentation was tested at 30, 60 or 90 minutes at 95°C.

The 10mM Tris fragmentation of Cre plasmid ds-cDNA resulted in average fragment size of 190 bp at 30 min and 42 bp at 60 min with 0% NMF, with 5% NMF fragments were average size of 60 bp after 30 min and with 10% NMF fragments were 40 bp after 30 min. In 5 mM Tris the Cre plasmid fragments were 170bp after 30 min and 40 bp after 60 min without NMF. Fragments were 30 bp after 30 min in 5% NMF and 23 bp after 30 min in 10% NMF. The ds cDNA (desalted and stored in 5 mM Tris-HCL ph 7.2 buffer) fragmentation in 5 mM Tris-HCL buffer without NMF gave average fragment sizes of 165, 75 and 40 bp after 30, 45 and 60 min of incubation at 65°C, respectively. With 5% NMF the fragment sizes were 320, 40 and 20 bp after 15, 30 or 45 min of incubation at 95°C, respectively. The ds cDNA fragmentation after desalting and exchanging buffer to 5 mM Tris-HCl, pH 7.2 took 30 to 45 min at 95°C, this improved rate of fragmentation may be the result of the removal of inhibitors to fragmentation that are present in the ds cDNA synthesis.

### Example 6 Cu(OP)₂ and Endo IV fragmentation of cDNA.

3µl of 100mM phosphate buffer, pH~7.0, 3 µl 10mM sodium ascorbate buffer and 3µl 50µM Cu(OP)₂ solution were added to 3µg DNA in 1 mM tris or phosphate buffer. Water was added to a final reaction volume of 30µl. The fragmentation reaction was incubated at 65°C for 10 min. The resulting fragments were cleaned up using a Biospin column according to the manufacturer's instructions. Deglycosylation and removal of 3' modifications was done by incubating about 33 µl of the cleaned up fragmentation reaction with 14 µl of 5x TdT buffer, 14µl of 25mM CoCl₂ and 6µl of Endo IV (2U/µl) and incubating at 37°C for 2 hours and at 65°C for 15 min. 3' end labeling with TdT and DLR was done by adding 1 µl of DLR and 4.4 µl of TdT (400U/µl) to the ~70 µl reaction mixture and incubating at 37°C for 1 hour, followed by the addition of 2 µl of 0.5M EDTA, pH 8. The labeled fragments were hybridized to an array using standard protocols.

### Example 7: Cu(OP)₂ and Endo IV fragmentation of cDNA with Phosphatase.

Mix 3µg cDNA, 1.5 mM Cu(OP)₂, 10mM H₂O₂ and incubate for 15 min at 37°C. Quench by adding EDTA to 10mM. Purify by bio-spin purification according to manufacturer's instructions. This purification step is optional and may be left out in some embodiments. Incubate at 95°C for 10 min. Add 5 Units Endo IV, 5 Units Shrimp Alkaline Phosphatase (optional) and incubate at 37°C for 16 hours then 65°C for 15 min. Standard TdT labeling conditions and hybridization to microarray.

### Example 8: Cu(OP)₂ and Endo IV fragmentation of single-stranded cDNA.

3 ug ss-cDNA was mixed in a solution of 10 mM phosphate pH ~7, 5 µM Cu(OP)₂, and 1 mM ascorbate and incubated at 65°C for10 or 15 min. EDTA was added to 0.5 mM and the products were either subjected to bio-spin purification or not. This was followed by an incubation at 95°C for 10 min. 12 units of Endo-IV was added and incubated at 37°C for 2 hours, followed by incubation at 65°C for 15 min to inactivate the Endo-IV. The products were subjected to a standard TdT/DLR labeling reaction and the labeled fragments were hybridized to a test array and a hybridization pattern was analyzed using standard conditions. The percent present calls for samples treated with the bio-spin column (bio-spin) or untreated (crude), compared to a DNase I treated sample, are shown in Fig. 12. The results are comparable to DNase I treatment, with the bio-spin percent present call being higher than crude and the 10 min fragmentation being higher than the 15 min fragmentation.

The observed fragmentation was rapid and reproducible and resulted in fragments that could be labeled by TdT after treatment with Endo IV. Higher levels of Endo IV may improve the labeling by reducing residual abasic sites and 3' ends that are blocked from TdT labeling by modifications.

### Example 9. Fe(EDTA) fragmentation of cDNA with Biotin-LC-hydrazide (Pierce, Rockford, IL) labeling.

137 µM ss-cDNA was incubated with 2.5 mM Fe-EDTA, and 53 mM H₂O₂ at 95°C for 30 min. The reaction was purified using a bio-spin column (Bio-Rad Laboratories). To label the fragments 2 µl of 5 mM Biotin-LC-hydrazide in DMSO was added and the reaction was incubated at 25°C for 70 min. The reaction was purified with a bio-spin column and analyzed by hybridization to a test array. Fragmentation was efficient and rapid and biotin incorporation was efficient.

### Example 10: Fragmentation of cDNA in Imidazole Buffer at High Temperature.

3 ug of single-stranded cDNA was incubated in 10mM imidazole-HCl buffer at 95°C for 15 minutes. The total volume was 30 µl. After cooling to room temp, 30 µl of fragmented ss cDNA was treated with 100U of Endo III. Reaction conditions were 1x Endonuclease III buffer supplemented with 100µg/ml BSA. The reaction was incubated at 37°C for 2 hours. The total volume was 60 µl. ARP-Biotin in DMSO:H2O (1:2) was added to the reaction mixture to a final concentration of 5mM. The total volume was 80 µl. The reaction mixture was incubated at 65°C for 30 minutes. The reaction mixture was then loaded on a Microcon YM-3 column. The column was centrifuged at 10,000g for 20 minutes. The flow through was discarded at 100µl of 10mM tris-HCl buffer was added. The buffer exchange was repeated 4 times. The results were analysed by PAGE using streptavidin to quantitate the amount of biotin incorporation. Endo III efficiently fragmented the abasic sites generated by imidazole (pH ~6.4 at 25°C) after incubation for 15 min. at 37°C and 45°C. Biotin-ARP reacted with the fragmented cDNA efficiently (> 95%) as judged by streptavidin gel shift assay.

### CONCLUSION

Many variations of the invention will be apparent to those of skill in the art upon reviewing the above description. The scope of the invention should be determined with reference to the appended claims.

## Claims

1. A method for fragmenting and labeling DNA comprising:
mixing the DNA in a reaction comprising a buffer that has a pH between 6 and 9 at a first temperature, wherein said first temperature is between 16 and 37°C, and less than 6 at a second temperature, wherein said second temperature is between 65 and 105°C, wherein the reaction is mixed at said first temperature;
incubating the reaction at the second temperature to generate a plurality of abasic sites in the DNA;
incubating the reaction under conditions that promote cleavage of abasic sites and optionally with a nuclease that has 3' phosphatase activity, wherein the condition that promotes cleavage of abasic sites comprises incubation with an apurinic/apyrimidinic (AP) endonuclease; and,
labeling the fragments in a reaction comprising TdT;
wherein the buffer comprises a buffer selected from the group consisting of Tris, imidazole and colamine.

2. The method of claim 1 wherein the first temperature is between 20 and 30°C and the second temperature is between 85 and 100°C.

3. The method of claim 1 wherein the second temperature is about 95°C and the reaction is incubated at the second temperature for about 30 to about 120 minutes.

4. The method of claim 1 wherein the buffer comprises EDTA.

5. The method of claim 1 wherein the buffer comprises acetate or citrate.

6. The method of claim 1 where the AP endonuclease is Endo IV or APE1.

7. The method of claim 1 or 3 wherein the reaction further comprises about 5 to 10% N-methylformamide.

8. A method for fragmenting and labeling a nucleic acid sample comprising DNA comprising:
generating a plurality of abasic sites in the DNA by a chemical method;
cleaving the phosphate backbone at a plurality of the abasic sites;
optionally removing modifications at the 3' ends of the fragments, wherein said modifications are moieties other than a 3' hydroxyl group; and
labeling the fragments with a detectable label;
wherein the nucleic acid sample is in a buffer solution comprising a buffer selected from the group consisting of Tris, imidazole and colamine and wherein said buffer solution has a pH between 6 and 9 at a temperature between 20 and 30°C and a pH less than 6 at a temperature greater than 85°C and wherein said chemical method comprises incubating the sample at a temperature greater than 85°C for at least 15 minutes.

9. The method of claim 1 or 8 wherein said buffer includes 10mM Tris-HCl and is selected such that it has a pH between 7.2 and 7.5 at 25°C.

10. The method of claim 8 wherein said step of cleaving the phosphate backbone comprises incubation with an AP endonuclease.

11. The method of claim 10 wherein said AP endonuclease is Endo IV.

12. The method of claim 8 wherein said step of cleaving the phosphate backbone is by heat and optionally by addition of base.

13. The method of claim 8 wherein the step of removing modifications from the 3' end comprises incubation with an AP endonuclease.

14. The method of claim 8 wherein the step of labeling with a detectable label comprises incorporation of biotin at the 3' end by terminal transferase addition.

## Patentansprüche

1. Ein Verfahren zum Fragmentieren und Markieren von DNA, umfassend:
das Mischen der DNA in einer Reaktion, welche einen Puffer umfasst, der bei einer ersten Temperatur, wobei die erste Temperatur zwischen 16 und 37°C liegt, einen pH zwischen 6 und 9 und bei einer zweiten Temperatur, wobei die zweite Temperatur zwischen 65 und 105°C liegt, von weniger als 6 aufweist, wobei die Reaktion bei der ersten Temperatur gemischt wird;
das Inkubieren der Reaktion bei der zweiten Temperatur, um eine Mehrzahl abasischer Stellen in der DNA zu erzeugen;
das Inkubieren der Reaktion unter Bedingungen, welche eine Spaltung von abasischen Stellen fördern, und gegebenenfalls mit einer Nuklease, die 3'-Phosphataseaktivität aufweist, wobei die Bedingung, welche eine Spaltung von abasischen Stellen fördert, die Inkubation mit einer apurinischen/apyrimidinischen (AP) Endonuklease umfasst; und
das Markieren der Fragmente in einer Reaktion, die TdT umfasst;
wobei der Puffer einen Puffer umfasst, der ausgewählt ist aus der Gruppe, bestehend aus Tris, Imidazol und Colamin.

2. Das Verfahren nach Anspruch 1, wobei die erste Temperatur zwischen 20 und 30°C liegt und die zweite Temperatur zwischen 85 und 100°C liegt.

3. Das Verfahren nach Anspruch 1, wobei die zweite Temperatur ca. 95°C beträgt und die Reaktion bei der zweiten Temperatur für ca. 30 bis ca. 120 Minuten inkubiert wird.

4. Das Verfahren nach Anspruch 1, wobei der Puffer EDTA umfasst.

5. Das Verfahren nach Anspruch 1, wobei der Puffer Acetat oder Citrat umfasst.

6. Das Verfahren nach Anspruch 1, wobei die AP-Endonuklease Endo IV oder APE1 ist.

7. Das Verfahren nach Anspruch 1 oder 3, wobei die Reaktion weiterhin ca. 5 bis 10% N-Methylformamid umfasst.

8. Ein Verfahren zum Fragmentieren und Markieren einer Nukleinsäureprobe, die DNA umfasst, umfassend:
das Erzeugen einer Mehrzahl von abasischen Stellen in der DNA durch ein chemisches Verfahren;
das Spalten des Phosphatrückgrats an einer Mehrzahl der abasischen Stellen;
das fakultative Entfernen von Modifikationen an den 3'-Enden der Fragmente, wobei die Modifikationen Anteile sind, die von einer 3'-Hydroxylgruppe verschieden sind; und
das Markieren der Fragmente mit einer nachweisbaren Markierung;
wobei die Nukleinsäureprobe in einer Pufferlösung vorliegt, die einen Puffer umfasst, der ausgewählt ist aus der Gruppe, bestehend aus Tris, Imidazol und Colamin, und wobei die Pufferlösung einen pH zwischen 6 und 9 bei einer Temperatur zwischen 20 und 30°C und einen pH von weniger als 6 bei einer Temperatur von mehr als 85°C aufweist und wobei das chemische Verfahren das Inkubieren der Probe bei einer Temperatur von mehr als 85°C für wenigstens 15 Minuten umfasst.

9. Das Verfahren nach Anspruch 1 oder 8, wobei der Puffer 10 mM Tris-HCl einschließt und so ausgewählt ist, dass dieser bei 25°C einen pH zwischen 7,2 und 7,5 aufweist.

10. Das Verfahren nach Anspruch 8, wobei der Schritt des Spaltens des Phosphatrückgrats die Inkubation mit einer AP-Endonuklease umfasst.

11. Das Verfahren nach Anspruch 10, wobei die AP-Endonuklease Endo IV ist.

12. Das Verfahren nach Anspruch 8, wobei der Schritt des Spaltens des Phosphatrückgrats durch Wärme und gegebenenfalls durch Zugabe einer Base stattfindet.

13. Das Verfahren nach Anspruch 8, wobei der Schritt des Entfernens von Modifikationen von dem 3'-Ende die Inkubation mit einer AP-Endonuklease umfasst.

14. Das Verfahren nach Anspruch 8, wobei der Schritt des Markierens mit einer nachweisbaren Markierung den Einbau von Biotin an dem 3'-Ende durch Zugabe einer terminalen Transferase umfasst.

## Revendications

1. Procédé de fragmentation et de marquage de l'ADN, comprenant :
le mélange de l'ADN dans une réaction comprenant un tampon qui possède un pH compris entre 6 et 9 à une première température, où ladite première température est comprise entre 16 et 37 °C, et inférieur à 6 à une seconde température, où ladite seconde température est comprise entre 65 et 105 °C, où la réaction est mélangée à ladite première température ;
l'incubation de la réaction à la seconde température afin de générer une pluralité de sites abasiques dans l'ADN ;
l'incubation de la réaction dans des conditions qui favorisent un clivage des sites abasiques et facultativement avec une nucléase qui présente une activité 3'-phosphatase, où la condition qui favorise le clivage des sites abasiques comprend une incubation avec une endonucléase apurinique/apyrimidique (AP) ; et,
le marquage des fragments dans une réaction comprenant une TdT ;
où le tampon comprend un tampon sélectionné dans le groupe comprenant le Tris, l'imidazole et la colamine.

2. Procédé selon la revendication 1, dans lequel la première température est comprise entre 20 et 30 °C et la seconde température est comprise entre 85 et 100°C.

3. Procédé selon la revendication 1, dans lequel la seconde température est d'environ 95°C et la réaction est incubée à la seconde température pendant environ 30 à environ 120 minutes.

4. Procédé selon la revendication 1, dans lequel le tampon comprend de l'EDTA.

5. Procédé selon la revendication 1, dans lequel le tampon comprend de l'acétate ou du citrate.

6. Procédé selon la revendication 1, dans lequel l'endonucléase AP est l'Endo IV ou APE1.

7. Procédé selon la revendication 1 ou 3, dans lequel la réaction comprend en outre du N-méthylformamide à environ 5 à 10 %.

8. Procédé de fragmentation et de marquage d'un échantillon d'acide nucléique comprenant de l'ADN, qui comprend :
la génération d'une pluralité de sites abasiques dans l'ADN par un procédé chimique ;
le clivage du squelette phosphate au niveau d'une pluralité des sites abasiques ;
l'élimination facultative des modifications aux extrémités 3' des fragments, où lesdites modifications sont des fragments autres qu'un groupe hydroxyle en 3' ; et
le marquage des fragments avec un marqueur détectable ;
où l'échantillon d'acide nucléique est dans une solution tampon comprenant un tampon sélectionné dans le groupe comprenant le Tris, l'imidazole et la colamine, et où ladite solution tampon possède un pH compris entre 6 et 9 à une température comprise entre 20 et 30 °C et un pH inférieur à 6 à une température supérieure à 85 °C, et où ledit procédé chimique comprend l'incubation de l'échantillon à une température supérieure à 85 °C pendant au moins 15 minutes.

9. Procédé selon la revendication 1 ou 8, dans lequel ledit tampon comprend du Tris-HCl 10 mM et est sélectionné de sorte qu'il possède un pH compris entre 7,2 et 7,5 à 25 °C.

10. Procédé selon la revendication 8, dans lequel ladite étape de clivage du squelette phosphate comprend une incubation avec une endonucléase AP.

11. Procédé selon la revendication 10, dans lequel ladite endonucléase AP est l'Endo IV.

12. Procédé selon la revendication 8, dans lequel ladite étape de clivage du squelette phosphate est réalisée par la chaleur et facultativement par l'addition d'une base.

13. Procédé selon la revendication 8, dans lequel l'étape d'élimination des modifications de l'extrémité 3' comprend une incubation avec une endonucléase AP.

14. Procédé selon la revendication 8, dans lequel l'étape de marquage avec un marqueur détectable comprend une incorporation de biotine à l'extrémité 3' par l'addition d'une transférase terminale.
